# EUROPEAN PATENT APPLICATION

(11) **EP 1 657 305 A1**
(43) Date of publication of application: **17.05.2006**
(21) Application number: 04771653.5
(22) Date of filing: 13.08.2004
(51) Int. Cl.: C12N 15/00, C12N 9/99, C12Q 1/48, C12Q 1/68

(54) **INTERACTION INHIBITORS, METHOD OF DETECTING INTERACTION INHIBITOR AND KIT FOR DETECTING INTERACTION INHIBITOR**

(30) Priority: 19.08.2003 JP 2003295204
(71) Applicant: Celestar Lexico-Sciences, Inc., Chiba-shi, Chiba 261-8501 (JP)
(72) Inventor: HIHARA, Satoshi, c/o Celestar Lexico-Sciences, Inc, Chiba-shi, Chiba 2618501 (JP); DOI, Hirofumi, c/o Celestar Lexico-Sciences, Inc., Chiba-shi, Chiba 2618501 (JP)
(74) Representative: HOFFMANN EITLE
(86) International application number: PCT/JP2004/011686
(87) International publication number: WO 2005/017146

(57) **Abstract**

The task is to provide protein interaction inhibitors and/or detection method for interaction inhibitors related to regulation of T cell activation and production of IL-2. The present invention provides inhibitors of interaction between PKC theta and KPNA1, and inhibitors of interaction between KPNA1 and NF-kappaB. Inhibitors of interaction is defined as an candidate compound demonstrating the inhibition of interaction by assaying whether interaction has occurred or not between 2 types of protein and the candidate compound under the conditions allowing interaction of each combination. The detection kit is equipped with samples providing proteins of combinations to be interacted.

SEQUENCE LISTING

<110> Celestar Lexico-Sciences, Inc.

<120> Interaction inhibitor, method for detecting interaction inhibitor and kit for
detecting interaction inhibitor

<130> PCLA-05171

<140> JP2003-295204
<141> 2003-8-19

<160> 11

<170> PatentIn version 3.1

<210> 1
<211> 23
<212> DNA
<213> Artificial

<220>
<223> Inventor: HIHARA, Satoshi
Inventor: DOI, Hirofumi

<220>
<223> primer(theta-N)

<400> 1
atgtcgccat ttcttcggat tgg 23

<210> 2
<211> 19
<212> DNA

<213>  Artificial

<220>
<223>  primer(theta-C)

<400>  2
tcaggatatc agccgctcc                                                    19

<210>  3
<211>  21
<212>  DNA
<213>  Artificial

<220>
<223>  primer(PKNA1-N)

<400>  3
atgaccaccc caggaaaaga g                                                 21

<210>  4
<211>  19
<212>  DNA
<213>  Artificial

<220>
<223>  primer(KPNA1-C)

<400>  4
tcaaagctgg aaaccttcc                                                    19

<210>  5
<211>  35
<212>  DNA

<213>  Artificial

<220>
<223>  primer(p50-N)

<400>  5
gcggccgcaa tggcagaaga tgatccatat ttggg                                35

<210>  6
<211>  30
<212>  DNA
<213>  Artificial

<220>
<223>  primer(p50-C)

<400>  6
ctcgagttac atggttccat gcttcatccc                                      30

<210>  7
<211>  30
<212>  DNA
<213>  Artificial

<220>
<223>  primer(p65-N)

<400>  7
gcggccgcaa tggacgaact gttccccctc                                     30

<210>  8
<211>  28
<212>  DNA

<213>  Artificial

<220>
<223>  primer(p65-C)

<400>  8
ctcgagttag gagctgatct gactcagc                                          28

<210>  9
<211>  706
<212>  PRT
<213>  human

<400>  9

Met Ser Pro Phe Leu Arg Ile Gly Leu Ser Asn Phe Asp Cys Gly Ser
1               5                   10                  15

Cys Gln Ser Cys Gln Gly Glu Ala Val Asn Pro Tyr Cys Ala Val Leu
            20                  25                  30

Val Lys Glu Tyr Val Glu Ser Glu Asn Gly Gln Met Tyr Ile Gln Lys
        35                  40                  45

Lys Pro Thr Met Tyr Pro Pro Trp Asp Ser Thr Phe Asp Ala His Ile
    50                  55                  60

Asn Lys Gly Arg Val Met Gln Ile Ile Val Lys Gly Lys Asn Val Asp
65                  70                  75                  80

Leu Ile Ser Glu Thr Thr Val Glu Leu Tyr Ser Leu Ala Glu Arg Cys
                85                  90                  95

Arg Lys Asn Asn Gly Lys Thr Glu Ile Trp Leu Glu Leu Lys Pro Gln
            100                 105                 110

Gly Arg Met Leu Met Asn Ala Arg Tyr Phe Leu Glu Met Ser Asp Thr
        115                 120                 125

Lys Asp Met Asn Glu Phe Glu Thr Glu Gly Phe Phe Ala Leu His Gln
    130                 135                 140

Arg Arg Gly Glu Ile Lys Gln Ala Lys Val His His Val Lys Cys His
145                 150                 155                 160

Glu Phe Thr Ala Thr Phe Phe Pro Gln Pro Thr Phe Cys Ser Val Cys
                165                 170                 175

His Glu Phe Val Trp Gly Leu Asn Lys Gln Gly Tyr Gln Cys Arg Gln
            180                 185                 190

Cys Asn Ala Ala Ile His Lys Lys Cys Ile Asp Lys Val Ile Ala Lys
        195                 200                 205

Cys Thr Gly Ser Ala Ile Asn Ser Arg Glu Thr Met Phe His Lys Glu
    210                 215                 220

Arg Phe Lys Ile Asp Met Pro His Arg Phe Lys Val Tyr Asn Tyr Lys
225                 230                 235                 240

Ser Pro Thr Phe Cys Glu His Cys Gly Thr Leu Leu Trp Gly Leu Ala
                245                 250                 255

Arg Gln Gly Leu Lys Cys Asp Ala Cys Gly Met Asn Val His His Arg
            260                 265                 270

Cys Gln Thr Lys Val Ala Asn Leu Cys Gly Ile Asn Gln Lys Leu Met
        275                 280                 285

Ala Glu Ala Leu Ala Met Ile Glu Ser Thr Gln Gln Ala Arg Cys Leu
    290                 295                 300

Arg Asp Thr Glu Gln Ile Phe Arg Glu Gly Pro Val Glu Ile Gly Leu
305                 310                 315                 320

Pro Cys Ser Ile Lys Asn Glu Ala Arg Pro Pro Cys Leu Pro Thr Pro
                325                 330                 335

Gly Lys Arg Glu Pro Gln Gly Ile Ser Trp Glu Ser Pro Leu Asp Glu
            340                 345                 350

Val Asp Lys Met Cys His Leu Pro Glu Pro Glu Leu Asn Lys Glu Arg
        355                 360                 365

Pro Ser Leu Gln Ile Lys Leu Lys Ile Glu Asp Phe Ile Leu His Lys
    370                 375                 380

Met Leu Gly Lys Gly Ser Phe Gly Lys Val Phe Leu Ala Glu Phe Lys
385                 390                 395                 400

Lys Thr Asn Gln Phe Phe Ala Ile Lys Ala Leu Lys Lys Asp Val Val
                405                 410                 415

Leu Met Asp Asp Asp Val Glu Cys Thr Met Val Glu Lys Arg Val Leu
            420                 425                 430

Ser Leu Ala Trp Glu His Pro Phe Leu Thr His Met Phe Cys Thr Phe
        435                 440                 445

Gln Thr Lys Glu Asn Leu Phe Phe Val Met Glu Tyr Leu Asn Gly Gly
    450                 455                 460

Asp Leu Met Tyr His Ile Gln Ser Cys His Lys Phe Asp Leu Ser Arg
465                 470                 475                 480

Ala Thr Phe Tyr Ala Ala Glu Ile Ile Leu Gly Leu Gln Phe Leu His
                485                 490                 495

Ser Lys Gly Ile Val Tyr Arg Asp Leu Lys Leu Asp Asn Ile Leu Leu
            500                 505                 510

Asp Lys Asp Gly His Ile Lys Ile Ala Asp Phe Gly Met Cys Lys Glu
        515                 520                 525

Asn Met Leu Gly Asp Ala Lys Thr Asn Thr Phe Cys Gly Thr Pro Asp
    530                 535                 540

Tyr Ile Ala Pro Glu Ile Leu Leu Gly Gln Lys Tyr Asn His Ser Val
545                 550                 555                 560

Asp Trp Trp Ser Phe Gly Val Leu Leu Tyr Glu Met Leu Ile Gly Gln
                565                 570                 575

Ser Pro Phe His Gly Gln Asp Glu Glu Glu Leu Phe His Ser Ile Arg
            580                 585                 590

Met Asp Asn Pro Phe Tyr Pro Arg Trp Leu Glu Lys Glu Ala Lys Asp
        595                 600                 605

Leu Leu Val Lys Leu Phe Val Arg Glu Pro Glu Lys Arg Leu Gly Val
    610                 615                 620

Arg Gly Asp Ile Arg Gln His Pro Leu Phe Arg Glu Ile Asn Trp Glu
625                 630                 635                 640

Glu Leu Glu Arg Lys Glu Ile Asp Pro Pro Phe Arg Pro Lys Val Lys
                645                 650                 655

Ser Pro Phe Asp Cys Ser Asn Phe Asp Lys Glu Phe Leu Asn Glu Lys
            660                 665                 670

Pro Arg Leu Ser Phe Ala Asp Arg Ala Leu Ile Asn Ser Met Asp Gln
        675                 680                 685

Asn Met Phe Arg Asn Phe Ser Phe Met Asn Pro Gly Met Glu Arg Leu
    690                 695                 700

Ile Ser
705

<210> 10
<211> 706
<212> PRT
<213> human

<400> 10

Met Ser Pro Phe Leu Arg Ile Gly Leu Ser Asn Phe Asp Cys Gly Ser
1               5                   10                  15

Cys Gln Ser Cys Gln Gly Glu Ala Val Asn Pro Tyr Cys Ala Val Leu
            20                  25                  30

Val Lys Glu Tyr Val Glu Ser Glu Asn Gly Gln Met Tyr Ile Gln Lys
        35                  40                  45

Lys Pro Thr Met Tyr Pro Pro Trp Asp Ser Thr Phe Asp Ala His Ile

    50                  55                  60

Asn Lys Gly Arg Val Met Gln Ile Ile Val Lys Gly Lys Asn Val Asp
65                  70                  75                  80

Leu Ile Ser Glu Thr Thr Val Glu Leu Tyr Ser Leu Ala Glu Arg Cys
                85                  90                  95

Arg Lys Asn Asn Gly Lys Thr Glu Ile Trp Leu Glu Leu Lys Pro Gln
            100                 105                 110

Gly Arg Met Leu Met Asn Ala Arg Tyr Phe Leu Glu Met Ser Asp Thr
        115                 120                 125

Lys Asp Met Asn Glu Phe Glu Thr Glu Gly Phe Phe Ala Leu His Gln
    130                 135                 140

Arg Arg Gly Ala Ile Lys Gln Ala Lys Val His His Val Lys Cys His
145                 150                 155                 160

Glu Phe Thr Ala Thr Phe Phe Pro Gln Pro Thr Phe Cys Ser Val Cys
                165                 170                 175

His Glu Phe Val Trp Gly Leu Asn Lys Gln Gly Tyr Gln Cys Arg Gln
            180                 185                 190

Cys Asn Ala Ala Ile His Lys Lys Cys Ile Asp Lys Val Ile Ala Lys

        195                 200                 205

Cys Thr Gly Ser Ala Ile Asn Ser Arg Glu Thr Met Phe His Lys Glu
    210                 215                 220

Arg Phe Lys Ile Asp Met Pro His Arg Phe Lys Val Tyr Asn Tyr Lys
225                 230                 235                 240

Ser Pro Thr Phe Cys Glu His Cys Gly Thr Leu Leu Trp Gly Leu Ala
                245                 250                 255

Arg Gln Gly Leu Lys Cys Asp Ala Cys Gly Met Asn Val His His Arg
            260                 265                 270

Cys Gln Thr Lys Val Ala Asn Leu Cys Gly Ile Asn Gln Lys Leu Met
        275                 280                 285

Ala Glu Ala Leu Ala Met Ile Glu Ser Thr Gln Gln Ala Arg Cys Leu
    290                 295                 300

Arg Asp Thr Glu Gln Ile Phe Arg Glu Gly Pro Val Glu Ile Gly Leu
305                 310                 315                 320

Pro Cys Ser Ile Lys Asn Glu Ala Arg Pro Pro Cys Leu Pro Thr Pro
                325                 330                 335

Gly Lys Arg Glu Pro Gln Gly Ile Ser Trp Glu Ser Pro Leu Asp Glu

            340                 345                 350

Val Asp Lys Met Cys His Leu Pro Glu Pro Glu Leu Asn Lys Glu Arg
        355                 360                 365

Pro Ser Leu Gln Ile Lys Leu Lys Ile Glu Asp Phe Ile Leu His Lys
    370                 375                 380

Met Leu Gly Lys Gly Ser Phe Gly Lys Val Phe Leu Ala Glu Phe Lys
385                 390                 395                 400

Lys Thr Asn Gln Phe Phe Ala Ile Arg Ala Leu Lys Lys Asp Val Val
                405                 410                 415

Leu Met Asp Asp Asp Val Glu Cys Thr Met Val Glu Lys Arg Val Leu
            420                 425                 430

Ser Leu Ala Trp Glu His Pro Phe Leu Thr His Met Phe Cys Thr Phe
        435                 440                 445

Gln Thr Lys Glu Asn Leu Phe Phe Val Met Glu Tyr Leu Asn Gly Gly
    450                 455                 460

Asp Leu Met Tyr His Ile Gln Ser Cys His Lys Phe Asp Leu Ser Arg
465                 470                 475                 480

Ala Thr Phe Tyr Ala Ala Glu Ile Ile Leu Gly Leu Gln Phe Leu His

                485                 490                 495

Ser Lys Gly Ile Val Tyr Arg Asp Leu Lys Leu Asp Asn Ile Leu Leu
            500                 505                 510

Asp Lys Asp Gly His Ile Lys Ile Ala Asp Phe Gly Met Cys Lys Glu
        515                 520                 525

Asn Met Leu Gly Asp Ala Lys Thr Asn Thr Phe Cys Gly Thr Pro Asp
    530                 535                 540

Tyr Ile Ala Pro Glu Ile Leu Leu Gly Gln Lys Tyr Asn His Ser Val
545                 550                 555                 560

Asp Trp Trp Ser Phe Gly Val Leu Leu Tyr Glu Met Leu Ile Gly Gln
                565                 570                 575

Ser Pro Phe His Gly Gln Asp Glu Glu Glu Leu Phe His Ser Ile Arg
            580                 585                 590

Met Asp Asn Pro Phe Tyr Pro Arg Trp Leu Glu Lys Glu Ala Lys Asp
        595                 600                 605

Leu Leu Val Lys Leu Phe Val Arg Glu Pro Glu Lys Arg Leu Gly Val
    610                 615                 620

Arg Gly Asp Ile Arg Gln His Pro Leu Phe Arg Glu Ile Asn Trp Glu

625                 630                 635                 640

Glu Leu Glu Arg Lys Glu Ile Asp Pro Pro Phe Arg Pro Lys Val Lys
                645                 650                 655

Ser Pro Phe Asp Cys Ser Asn Phe Asp Lys Glu Phe Leu Asn Glu Lys
            660                 665                 670

Pro Arg Leu Ser Phe Ala Asp Arg Ala Leu Ile Asn Ser Met Asp Gln
        675                 680                 685

Asn Met Phe Arg Asn Phe Ser Phe Met Asn Pro Gly Met Glu Arg Leu
    690                 695                 700

Ile Ser
705

<210>  11
<211>  14
<212>  DNA
<213>  Artificial

<220>
<223>  enhancer

<400>  11
tggggacttt ccgc                                                    14

## Description

### [FIELD OF THE INVENTION]

The present invention relates to inhibitors of protein-protein interaction, a detection method for inhibitors of the interaction, and a detection kit for inhibitors of the interaction in the signaling pathway related to the regulation of T cell activity.

### [Background of Invention]

PKC (protein kinase C) family are serine/threonine kinases activated by specific binding to various lipid messengers such as calcium ion, phospholipids, fatty acids, phorbol ester and diacylglycerol and regulate growth and proliferation of cells. PKC family are divided into three families based on the difference of regulatory domain, "Conventional" or "Classical", "Novel", and "Atypical". The conventional/classical family includes three types: alpha, beta and gamma. The novel family includes four types: delta, epsilon, eta and theta. The atypical family includes two types: zeta and iota.

PKC theta is a member of PKC family and belongs to a novel calcium ion-independent type. One of known functions of PKC theta is activation of T cells. Other PKCs are also expressed in T cells, but it is considered that only PKC theta among PKCs contributes to T cells.

T cell which recognized antigen produces proliferation factor IL-2 (interleukin 2), and expresses IL-2 receptor, and proliferates in an IL-2-dependent manner. In PKC theta-knockout mouse, above T cell activation in response to external stimuli does not occur, and IL-2 production decreases (for example, Non-patent Literature 1). In IL-2 gene-knockout mouse is known to develop inflammatory bowel disease and hemolytic anemia (for example, Non-patent Literature 2).

It is also known that transcription factors such as NF-kappa B play an important role in regulation of transcription activity of IL-2 gene.

[Non-patent Literature 1] Christopher W. A. et al., (2002) Current Opinion in Immunology 12:323-330 [Non-patent Literature 2] Horak I., (1995) Clinical Immunology and Immunopathology Sep; 76(3 Pt 2): S172-3

### [Disclosure of the Invention]

### [Problems to be Resolved by the Invention]

T cell and IL-2 play a very important role in body's physiological function, especially in immune system. But specific signaling pathways related to the regulation of T cell activity and production of IL-2 are not still well understood. These signaling pathways related to the regulation of T cell activity and production of IL-2 is predicted to be wide-ranged and complex. Although identifying the signaling pathway requires the knowledge of which substance being involved at which step, it is not easy even identifying substances involved in the signaling pathways, because the many substances may be involved in the signaling pathways related to the regulation of T cell activity, and these substances may function in very small amount or their stability may be low.

On the other hand, elucidation of the signaling pathways related to T cell or IL-2 will significantly contribute to the prevention or treatment of diseases related to the abnormality of T cells or IL-2, and to the development of relevant drugs. Elucidation of the signaling pathways will lead to the exploitation of new drug discovery target. If the new combined interaction between proteins involved in the signaling pathway and the drugs inhibiting the newly discovered protein interaction were discovered, the drugs can regulate the interaction. That means the drugs can be used in the prevention or treatment of the diseases related to the signaling pathways. Thus, discovery of the new combination of protein interaction in the signaling pathway provides the novel drug discovery target, prompting the development of new drugs with different mechanism of action from the existing ones.

The present invention was done in view of above-mentioned situation, and an object of the present invention is to provide inhibitors of protein-protein interaction related to the regulation of T cell activity or production of IL-2, detection methods for inhibitors of the interaction and detection kits for inhibitors of the interaction.

### [Means of Solving the Problems]

In view of the problems mentioned above, the present inventors studied factors which were predicted to be related to T cells or IL-2, searching factors associated with PKC theta and/or NF-kappa B, which are the important factors in the signaling pathways of regulation of T cell activity or production of IL-2. As the result, the present inventors found out the new combined interaction associated with PKC theta and NF-kappa B, and perfected the present invention based on such findings. The present invention provides the following inhibitors of interaction, detection methods for inhibitors of the interaction, detection kits for inhibitors of the interaction and drug discovery targets.

[1] An inhibitor of protein-protein interaction inhibiting interaction between PKC theta and KPNA1.
[2] An inhibitor of protein-protein interaction inhibiting interaction between KPNA1 and NF-kappa B.
[3] An inhibitor of interaction between PKC theta and KPNA1, said inhibitor being obtained by examining whether the interaction between PKC theta and KPNA1 occurs in the presence of PKC theta, KPNA1, and candidate compounds under the conditions allowing interaction between PKC theta and KPNA1, and selecting a candidate compound showing inhibition of the interaction.
[4] The inhibitor as set forth in [3], wherein said PKC theta is a kinase having a constant phosphorylation activity.
[5] An inhibitor of interaction between KPNA1 and NF-kappa B, said inhibitor being obtained by examining whether the interaction between KPNA1 and NF-kappa B occurs in the presence of KPNA1, NF-kappa B, and candidate compounds under the conditions allowing interaction between KPNA1 and NF-kappa B, and selecting a candidate compound showing inhibition of the interaction.
[6] A method for detecting an inhibitor of interaction between PKC theta and KPNA1, said method comprising the step of examining whether the interaction between PKC theta and KPNA1 occurs in the presence of PKC theta, KPNA1, and candidate compounds under the conditions allowing interaction between PKC theta and KPNA1, to detect a candidate compound showing inhibition of the interaction as the inhibitor.
[7] The inhibitor as set forth in [6], wherein said PKC theta is a kinase having a constant phosphorylation activity.
[8] A method for detecting an inhibitor of interaction between KPNA1 and NF-kappa B, said method comprising the step of examining whether the interaction between KPNA1 theta and NF-kappa B occurred in the presence of KPNA1, NF-kappa B, and candidate compounds under the conditions allowing interaction between KPNA1 and NF-kappa B, to detect candidate compounds showing inhibition of the interaction as the inhibitor.
[9] A detection kit for an inhibitor of interaction between PKC theta and PKNA1, said kit comprising PKC theta supplying sample and KPNA1 supplying sample.
[10] The detection kit for an inhibitor as set forth in [9], wherein said PKC theta supplying sample is a vector containing polynucleotide coding PKC theta, and said KPNA1 supplying sample is a vector containing polynucleotide coding KPNA1.
[11] The kit as set forth in [9] or [10], wherein said PKC theta is a kinase having a constant phosphorylation activity.
[12] A detection kit for an inhibitor of interaction between KPNA1 and NF-kappa B, said kit comprising KPNA1 supplying sample and NF-kappa B supplying sample.
[13] The detection kit for an inhibitor as set forth in [12], wherein said KPNA1 supplying sample is a vector containing polynucleotide coding KPNA1, and said NF-kappa B supplying sample is a vector containing polynucleotide coding NF-kappa B.
[14] A method for developing a medicine characterized by targeting the interaction between PKC theta and KPNA1 for drug discovery.
[15] A method for developing a medicine characterized by targeting the interaction between KPNA1 and NE-kappa B for drug discovery.

### [Effect of the Invention]

With the present invention, inhibitors of interaction involving PKC theta and NE-kappa B, detection methods of the interaction, and detection kits will be provided. The present invention also provides drug discovery targets. Since PKC theta and NF-kappa B are important factors in signaling biological information Such as regulation of T cell activity and production of IL-2, the present invention will contribute to biotechnology industries including pharmaceutical industry.

### [Brief Description of Drawings]

Figure 1 shows that PKC theta binding with KPNA1 in cells.
Figure 2 shows that KPNA1 was phosphorylated by PKC theta.
Figure 3 shows that KPNA1 binds with NF-kappaB constitutive protein p50 and p65 in cells.
Figure 4 is a schematic view of the part of the left side electrophoresis image of Figure 1.
Figure 5 is a schematic view of the part of the electrophoresis image of Figure 2.
Figure 6 is a schematic view of the part of the left side electrophoresis image of lanes 1 and 2 in Figure 3.
Figure 7 shows the experimental results in Example 4.

### [The Best Mode to Perform the Invention]

The present invention will be further illustrated by the following exemplification. As well, the descriptions in various experiment manuals such as Molecular Cloning: A LABORATORY MANUAL, 3rd edition, Cold Spring Harbor Laboratory Press, Cold Spring Harbor, New York (2001) are referred when performing biochemical or genetic engineering procedure of the present invention.

(1) Inhibitors of interaction [0015] The inhibitor of the present invention is a substance inhibiting protein-protein interaction related to the regulation of T cell activity or production of IL-2, and includes two embodiments that are an inhibitor of interaction between PKC theta and KPNA1 and an inhibitor of interaction between KPNA1 and NF kappa B.

(1-1) Inhibitors of interaction between PKC theta and KPNA1
One embodiment of the present invention is an inhibitor which inhibits interaction between PKC theta and KPNA1.

PKC theta effects on T cell activation and is essential for IL-2 production. It is also required induction of IL-2 gene transcription by transcription factors such as NF-kappa B for IL-2 production. However, it had been unclear by which signaling pathway PKC theta induces transcription of IL-2 gene by transcription factors such as NF-kappa B. Although it is known that activation of NF-kappa B as a transcription factor is regulated by migration of its intracellular localization to the nucleus, the pathway related to the nuclear translocation was unknown. Meanwhile, KPNA1 recognizes protein functioning in the nucleus such as transcription factors, and helps to transport such proteins from the cytoplasm to the nucleus. The present inventors confirmed that PKC theta and KPNA1 interact, that PKC theta phosphorylates substrate KPNA1, and that KPNA1 and NF-kappa B interact. These findings revealed that KPNA1 which is responsible for nuclear translocation plays a role in the signaling pathway from PKC theta to NF-kappa B activation.

Thus, substances inhibiting interaction between PKC theta and KPNA1 are likely to regulate T cell activation and IL-2 production. In other words, substances inhibiting interaction between PKC theta and KPNA1 will be strong drug candidates for prevention and/or treatment of diseases related to the regulation of T cell activation or production of IL-2. Since there had been no findings suggesting that PKC theta and KPNA1 are related directly in the signaling pathway, even a primary concept that the above mentioned inhibitor of the present embodiment may be such a strong drug candidates for prevention and/or treatment of diseases related to the regulation of T cell activation or production of IL-2 was not exist. The present invention also provides totally new specific target for drug discovery by presenting the new combined interaction between PKC theta and KPNA1.

The inhibitor of the present embodiment may be a substance which inhibits interaction between PKC theta and PKNA 1, but otherwise not be limited. For example, the inhibitor of the present embodiment is not limited by materials, and it may be biological material such as polynucleotide and protein, or it may be chemicals such as organic or inorganic substance.

More specifically, the inhibitor of the present embodiment is exemplified as a compound which demonstrates inhibition of interaction by examining whether the interaction between PKC theta and KPNA1 occurred in the presence of PKC theta, KPNA1, and candidate compounds under the conditions allowing interaction between PKC theta and KPNA1.

(1-2) Inhibitors of interaction between KPNA1 and NF-kappa B
Another embodiment of the present invention is an inhibitor which inhibits interaction between KPNA1 and NF-kappa B.

As described above, the present inventors revealed that KPNA1 interacts with PKC theta, which effects on T cell activation, and is a substrate which is phosphorylated by PKC theta. The present inventors further revealed that NF-kappa B interacts with KPNA1 which is responsible for nuclear translocation of protein. NF-kappa B is a factor which activates transcription of IL-2 gene when translocated to the nucleus.

Thus, substances inhibiting interaction between KPNA1 and NF-kappa B are likely to repress IL-2 production. In other words, substances inhibiting interaction between KPNA1 and NF-kappa B will be strong drug candidates for prevention and/or treatment of diseases related to the regulation of T cell activation or abnormal production of IL-2. Since there had been no findings suggesting that KPNA1 and NF-kappa B are related directly in the signaling pathway, even a primary concept that the above mentioned inhibitor of the present embodiment may be such a strong drug candidates for prevention and/or treatment of diseases related to the regulation of T cell activation or production of IL-2 was not exist. The present invention also provides totally new specific target for drug discovery by presenting the new combined interaction between KPNA1 and NF-kappa B.

The inhibitor of the present embodiment may be a substance which inhibits interaction between KPNA1 and NF-kappa B, but otherwise not be limited. For example, the inhibitor of the present embodiment is not limited by materials, and it may be biological material such as polynucleotide and protein, or it may be chemicals such as organic or inorganic substance.

More specifically, the inhibitor of the present embodiment is exemplified as a compound which demonstrates inhibition of interaction by examining whether the interaction between KPNA1 and NF-kappa B occurred in the presence of LPNA1, NF-kappa B, and candidate compounds under the conditions allowing interaction between KPNA1 and NF-kappa B.

(2) Detection methods for the interaction inhibitors
The detection method for the interaction inhibitor of the present invention is detecting substances inhibiting protein-protein interaction related to the regulation of T cell activity or production of IL-2, and includes two embodiments of detection methods for inhibitors of interaction between PKC theta and KPNA1 and detection methods for inhibitors of interaction between KPNA1 and NF-kappa B.

(2-1) Detection methods for inhibitors of interaction between PKC theta and KPNA1
The present invention will be further illustrated by the following exemplification of the detection methods for inhibitors of interaction between PKC theta and KPNA1.

One embodiment of the present invention examines whether interaction between PKC theta and KPNA1 occurs in the presence of PKC theta, KPNA1, and candidate compounds under the conditions allowing interaction between PKC theta and KPNA1. Setting up the conditions allowing interaction between PKC theta and KPNA1 is the premise for precise detection for inhibition of protein interaction by candidate inhibitors. Thus it is only required to be conditions allowing protein interaction, for example, in vitro or in vivo. Preferably, the conditions should allow each protein to maintain the configuration of interface region in protein interaction.

For an example, liquid solvent to which protein is added provides a field which allows protein-protein interaction to occur. Preferable liquid solvent is aqueous solution preferably at ambient temperature, or more preferably at 30-37°C. The pH is adjusted to preferably neutral or more preferably between 6.5 and 8.5. Liquid solvent prepared in this way may be added other supplementary components such as a proper buffering agent to maintain the above pH.

The gene sequences of PKC theta and KPNA1 have become public knowledge. So these proteins can be genetic engineeringly prepared by transduction and expression in given host cells, and allowed to interact. For example, genes of PKC theta and KPNA1 can be obtained by creating probes based on the sequence described in a sequence database and isolated from cDNA library, or creating primers and amplifying with PCR from cDNA library. The cDNA library containing PKC theta and KPNA1 is commercially available.

In the present experimental system, it is preferable to employ PKC theta having a constant phosphorylation activity. "Having a constant phosphorylation activity" refers to the ability to constantly exhibit phosphorylation activity under an ordinary biochemical condition in which the three-dimensional structure of the protein is preserved. Protein having such an ability is different from those whose phosphorylation activity switches on and off depending on an external factor. As used herein, the phosphorylation activity refers to an activity to catalyze phosphorylation. In the method for detecting the inhibitor of interaction between PKC theta and KPNA 1, the interaction of PCK theta and KPNA1 may be detected in terms of phosphorylation of KPNA1. Therefore, the employment of inactivated PCK theta might cause false negative results. Consequently, employment of the aforementioned PCK theta having constant phosphorylation activity can improve reliability of the detection method.

Examples of the kinase having constant phosphorylation activity may include the protein having the amino acid sequence represented by SEQ ID NO:9. The amino acid sequence of SEQ ID NO:9 was the sequence obtained by replacing alanine with glutamic acid in a certain original sequence at 148th position from the N-terminus thereof. The substitution of the 148th amino acid residue with glutamic acid results in preservation of the switch-on state of the phosphorylation activity. In the present invention, it is also possible to employ protein that is substantially the same as the protein having the amino acid sequence represented by SEQ ID NO:9. That is, it may also possible to employ a protein having the 148th residue being glutamic acid, and having a sequence resulting from the substitution, deletion, insertion, addition or inversion of one or several amino acid residues in the region other than the 148th glutamic acid of the amino acid sequence of SEQ ID NO:9. As used herein, "several" means the acceptable range of number of mutation which does not cause significant damage on the constant phosphorylation activity. Specific example of the range in numbers may be 2 to 50, preferably 2 to 30, and more preferably 2 to 10.

Introduction of the mutation at the 148th amino acid residue from alanine to glutamic acid may be achieved by modifying the basic sequence of the gene encoding the protein by, e.g., a site-specific mutation method so that the specific amino acid is replaced.

PKC theta, KPNA1 and a candidate compound are put together under the conditions allowing interaction between PKC theta and KPNA1. In other words, PKC theta, KPNA1 and a candidate compound are brought into contact. Specifically, PKC theta, KPNA1, and a candidate compound can be added to the liquid solvent prepared as above. Alternatively, a candidate compound can be introduced to cells expressing PKC theta and KPNA1, or a candidate compound can be expressed in the same cells.

A candidate compound which shows interaction inhibition is obtained by examining inhibition of interaction as described above. It is easily identified whether interaction is inhibited or not by comparing with a proper control. A proper control is exemplifies as a system confirming the experimental system functions normally, as well as a system pre-established to allow protein interaction to occur or not to occur demonstrating that an unknown candidate compound inhibits protein interaction or not with comparison. Various labeling material such as luminescent material, fluorescent material, coloring material, radioactive material or marker gene is used for identification. The labeling material is measured qualitatively or quantitatively, and the candidate compounds can be judged as an inhibitor or not by difference of the measurements.

Some assays to detect protein interaction have been already known. Specifically, immunoprecipitation, Far-Western, gel filtration, two-hybrid, energy transfer or surface plasmon resonance are some examples. For the detection of the present invention, an experimental system should be established to detect inhibitors with a proper control sample and a detection method for protein interaction as mentioned above.

(2-2) Detection methods for inhibitors of interaction between KPNA1 and NF-kappa B
In order to detect inhibitors of interaction between KPNA1 and NF-kappa B, it is only necessary to change the above combination of PCK theta and KPNA1 to the combination of KPNA1 and NF-kappa B. Assay is performed to see whether the interaction between KPNA1 and NF-kappa B occurred in the presence of KPNA1, NF-kappa B, and candidate compounds under the conditions allowing interaction between KPNA1 and NF-kappa B. And the candidate compounds which inhibit interaction are detected as inhibitors. Other preferable conditions are the same in the detection method of inhibitors of interaction between PKC theta and KPNA1 as described above in (2-1).

(3) Detection kits for interaction inhibitors
The detection kit for the interaction inhibitor of the present invention is the assay kit for detection of substances inhibiting protein-protein interaction related to the regulation of T cell activity or production of IL-2, and includes two embodiments of detection kits for inhibitors of interaction between PKC theta and KPNA1 and detection kits for inhibitors of interaction between KPNA1 and NF-kappa B. For forms of sample supplier can be purified protein as well as polynucleotides coding these proteins. By incorporating these polynucleotides into the proper vector, these proteins can be easily provided to experiments by genetic engineering technology. Furthermore, as long as the interaction and function of these proteins is retained, beta-galactosidase, glutathione-S-transferase, or peptide tag such as His tag, myc tag or FLAG tag can be added to the N-terminus and/or the C-terminus of these polynucleotides.

(3-1) Detection kits for inhibitors of interaction between PKC theta and KPNA1
The kit of the present invention will be further illustrated by the following exemplification of the detection kits for inhibitors of interaction between PKC theta and KPNA1. One embodiment of the detection kit of the present invention comprises a PKC theta supplying sample and a KPNA1 supplying sample. The PKC theta supplying sample, for example, can be purified PKC theta protein or polynucleotide coding PKC theta. One preferable embodiment of the assay kit consists of a vector containing polynucleotide coding PKC theta and a vector containing polynucleotide coding KPNA1.

The detection kit can adopt various formats for an expression vector by combining type of vector (i.e. plasmid), promoter, and selection marker. Examples thereof are as follows.

For the vector, for example, plasmids derived from E. coli (i.e. pBR3222, pBR325, pUC12, pUC13 or commercially available pBT Vector or pTRG Vector (Stratagene)), plasmids derived from yeast (i.e. YEp24 or YCp50), bacteriophage such as lambda phage, animal virus such as retrovirus, vaccinia virus or baculovirus, as well as pAl-11, pXT1, pRc/CMV, pRc/RSV or pcDNAI/Neo can be used. And plasmid suitable for Bacillus subtilis is pUB110, pTP5 or pC194.

Any promoter will do as long as it is appropriate for the host cells expressing the gene. For example, for Escherichia host cells, trp promoter, lac promoter, recA promoter, lambda PL promoter, lpp promoter or T7 promoter can be used. For Bacillus host cells, SPO1 promoter, SP02 promoter or penP promoter can be used. For yeast host cells, PHO5 promoter, PGK promoter, GAP promoter or ADH promoter can be used. For insect host cells, polyhedrin promoter or P10 promoter can be used. For animal host cells, SR alpha promoter, SV40 promoter, HIV-LTR promoter, CMC (cytomegalovirus) promoter or HSV-TK promoter can be used.

Expression vector preferably has a multicloning site from the aspect of easy-handling in recombination process. In addition to mentioned above, a selection marker, an enhancer, a splicing signal, a polyA additional signal, a SV40 replication origin (hereinafter abbreviated to SV40ori in some cases) or a terminator can be incorporated into the expression vector as required. Examples of the selection marker may include an ampicillin-resistant gene (which can also work as a carbenicillin-resistant gene, and which may be abbreviated hereinbelow as Amp^{r}), a chloramphenicol-resistant gene (which may be abbreviated hereinbelow as Cam^{r}), a tetracycline-resistant gene (which may be abbreviated hereinbelow as Tet^{r}), a dihydrofolate reductase (which may be abbreviated hereinbelow as dhf^{r}) gene (methotrexate resistant), and a neomycin-resistant gene (which may be abbreviated hereinbelow as Neo^{r}, G418 resistant). If necessary, a signal sequence suitable for the relevant host cells may be added to the vector. Examples of the signal sequence for use may include a PhoA signal sequence and an OmpA signal sequence for genus Escherichia hosts; an alpha-amylase signal sequence and a subtilicin signal sequence for genus Bacillus hosts; an MF alpha signal sequence and an SUC2 signal sequence for yeast hosts; and an insulin signal sequence, an alpha-interferon signal sequence, and an antibody molecule signal sequence for animal cell hosts.

The kit of the present invention may contain host cells suitable for an expression vector. Host cells may be, for example, bacterial cells such as streptocicci, staphylococci, Escherichia coli, Streptomyces or Bacillus; fungal cells such as yeast or Aspergillus; insect cells such as Drosophila S2 or Spodoptera Sf9; animal cells such as CHO, COS, HeLa, C127, 3T3, BHK, HEK293, Bows melanoma cells or hematocyte; or plant cells. For the detection of inhibitors of the present invention, preferably yeast cells, E. coli cells, Bacillus cells or mammalian cells are recommended as host cells.

Introduction of expression vector into host cells is performed according to the standard experiment manuals such as Davis et.al. BASIC METHODS IN MOLECULAR BIOLOGY (1986) and Sambrook et.al. Molecular Cloning: A LABORATORY MANUAL, 3rd ed. Cold Spring Harbor Laboratory Press, Cold Spring Harbor, New York (2001). Particularly, for example, calcium phosphate transfection, DEAE-dextran mediated transfection, microinjection, cation lipid mediated transfection, electroporation, transduction, scrape loading, ballistic introduction or infection can be used.

Cultivation of transformant will be adjusted according to the type of the host. There are many types of host, here are some examples. When cultivating transformants in Escherichia host or Bacillus host, the culture medium can be either liquid medium or agar medium, containing necessary source of carbon and nitrogen, mineral, etc for growth of transformants. As carbon source, glucose, dextrin, soluble starch or sucrose; as nitrogen source, inorganic or organic material such as ammonium salts, nitrates, corn steep liquor, peptone, casein, meat extract, soybean cake or potato extract; as mineral, calcium chloride, sodium dihydrogen phosphate or magnesium chloride can be used. Also, yeast extract, vitamins and/or growth factors can be added. The pH of the medium should be between 5 and 8. The specific example of the favorable medium for Escherichia is LB medium containing yeast extract, tryptone and salt (NaCl). Into this medium, a inducer such as isopropyl 1-thio-beta-D-galactoside can be added to make promoter work effectively when necessary. If the host is Escherichia, incubation is generally carried out at 15-43°C for about 3-24 hours, and aeration and/or agitation will be added when necessary.

When cultivating transformant in yeast host, the culture medium should be Burkholder minimum medium or SD medium containing 0.5% of casamino acid. The pH of the medium should be adjusted between 5 and 8. Incubation is generally carried out at 20-35 °C for about 24-72 hours, and aeration and/or agitation will be added when necessary.

When cultivating transformant in insect cell host or insect host, the culture medium should be Grace's Insect Medium (Grace, T. C. C., Nature, 195, 788 (1962)) containing additives such as 10% of inactivated bovine serum if necessary. The pH of the medium should be adjusted between 6.2 and 6.4. Incubation is generally carried out at 27°C for about 3-5 days, and aeration and/or agitation will be added when necessary.

When cultivating transformant in animal cell host, the culture medium should be MEM medium containing 5-20% of fetal bovine serum, DMEM medium, RPMI 1640 medium (The Journal of the American Medical Association, 199, 519 (1967)), 199 medium (Proceeding of the Society for the Biological Medicine, 73, 1 (1950)). The pH of the medium should be adjusted between 6 and 8. Incubation is generally carried out at 30-40°C for about 15-60 hours, and aeration and/or agitation will be added when necessary. If necessary, CO₂ concentration should be adjusted.

The kit of the present invention may be provided with other detection means which are used to perform the detection of the present invention described above. For example, kits of this embodiment may contain reagents which are used to detect inhibition the protein interaction described above. For as reagents, labeling reagents which is measurable qualitatively or quantitatively such as luminescent material, fluorescent material, coloring material or radioactive material, antibody reagents for specific detection of the objective substance, auxiliary substances such as pH adjuster, buffering agent and base material can be selected and provided accordingly. The kit of the present invention may also be provided with a container for the detection assay, restriction enzymes and media for host cell cultivation.

(3-2) Detection kits for inhibitors of interaction between KPNA1 and NF-kappa B
For the detection kit for the inhibitor of interaction between KPNA1 and NF-kappa B, it is only necessary to change the above combination of PCK theta and KPNA1 to the combination of KPNA1 and NF-kappa B. That is, another embodiment of the kit of the present invention includes a KPNA1 supplying sample, a NF-kappa B supplying sample and assay method for the interaction between KPNA1 and NF-kappa B. One preferable form consists of a vector containing polynucleotide coding KPNA1 and a vector containing polynucleotide coding NF-kappa B. The other components are the same as those in detection kits for inhibitors of interaction between PKC theta and KPNA1 as set forth in (3-1) .

### [Examples]

The present invention will be described in more detail by the following examples, but will not be restricted thereto.

(Example 1) Analysis of interaction of PKC theta and KPNA1
The in vivo binding assay was performed to confirm experimentally whether PKC theta and KPNA1 interacted or not.

1-1) Construction of mammalian cell expression plasmid of PKC theta
The cDNA coding amino acid sequence for human PKC theta (National Center Biotechnology Information: NCBI; accession number NP_006248) was obtained by performing PCR with human skeletal muscle cDNA library (Takara Bio) as a template. The primer sequence used in PCR, theta-N and theta-C is shown in Sequence ID numbers 1 and 2 respectively in Sequence List. The resultant DNA fragment was inserted into mammalian cell expression vector pcDNA3.1/myc-HisB (Invitrogen). Thus, PKC theta-myc-His/pcDNA3.1, the plasmid which can express human PKC theta as protein tagged with myc-His at C-terminus (hereinafter called PKC theta-myc-His) in mammalian cells was constructed.

1-2) Construction of mammalian cell expression plasmid of KPNA1
The cDNA coding amino acid sequence for human KPNA1 (NCBI; accession number AAH03009) was obtained by performing PCR with human thymus cDNA library (Takara Bio) as a template. The sequence of primers (KPNA1-N and KPNA-C) used in PCR, is shown in Sequence ID numbers 3 and 4 respectively in Sequence List. The resultant DNA fragment was inserted into mammalian cell expression vector pCMV Tag 2C (Stratagene). Thus, FLAG-KPNA1/pCMV, the plasmid which can express human KPNA1 as protein tagged with FLAG at N-terminus (hereinafter called FLAG-KPNA1) in mammalian cells was constructed.

1-3) In vivo binding assay of PKC theta and KPNA1
The experiment was carried out by transfecting the expression vectors described above, PKC theta-myc-His/pcDNA3.1 and FLAG-KPNA1/pCMV into human embryonic kidney cell line, HEK293T cells.

First, 4 x 10⁵ of HEK293T cells were seeded onto 6 cm dishes, incubated at 37°C/5% CO₂ for overnight, and transfected by using FuGENE(Roche Diagnostics). At this time, two types of transfection were performed: 2 micrograms each of PKC theta-myc-His/pcDNA3.1 and FLAG-KPNA1/pCMV (combination 1), and as a negative control of 2 micrograms each of pcDNA3.1/myc-HisB (vector only) and FLAG-KPNA1/pCMV (combination 2).

After transfection, each protein was expressed transiently by further incubation at 37°C/5% CO₂ for two more days. Then the cells were washed with ice-cooled D-PBS (Invitrogen), and suspended in 0.5 ml of Cell Lysis Buffer (20 mM Tris-HC1, pH7.5/ 150 mM NaCl/ 1 mM Na₂EDTA/ 1 mM EGTA/ 1% Triton / 2.5 mM sodium pyrophosphate/ 1 mM beta-glycerophosphate/ 1 mM Na₃VO₄/ 1 microgram/ml Leupeptin/ 1 mM PMSF), and left to stand for 30 minutes on ice. Then the cell extract was obtained by collecting supernatant with centrifugation at 14 krpm for 10 minutes at 4°C. Ten microlillters of agarose conjugated normal mouse IgG (SantaCruz) was added to this collected cell extract, and mixed by inverting tube. The supernatant was collected by centrifugation (Pre-clean).

Ten microlitters of the anti-FLAG M2 agarose affinity gel (Sigma) was added onto the supernatant (immunoprecipitation of FLAG-KPNA1), and mixed by inverting tube for 2 hours at 4°C. The agarose was collected by centrifugation. The agarose was washed with 0.5 ml of Cell Lysis Buffer for four times. SDS sample buffer was added to the agarose and the mixture was boiled for 5 minutes. The supernatant was separated by SDS-PAGE. Immunoprecipitation of FLAG-KPNA1 was confirmed by western blotting with anti-FLAG M3 Monoclonal Antibody (Sigma). And co-precipitation of PKC theta-myc-His was detected by western blotting using c-Myc(9E10) Monoclonal Antibody (SantaCruz) . ECL plus western blotting kit (Amersham biosciences) was used for detection.

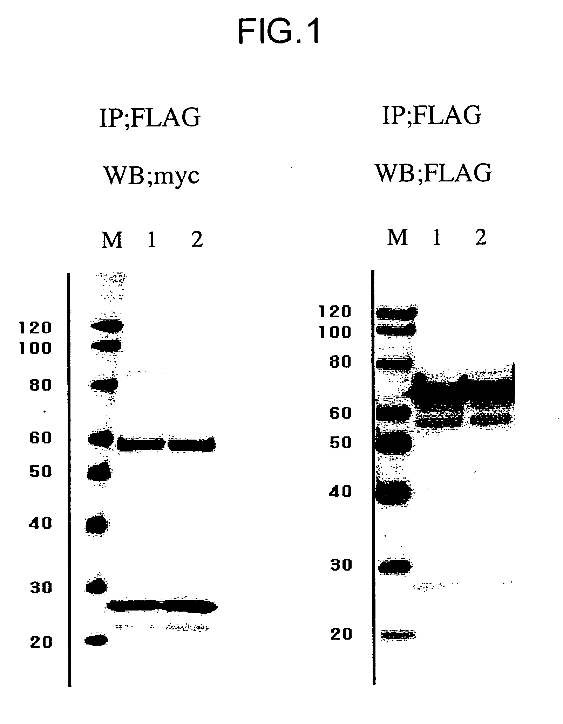

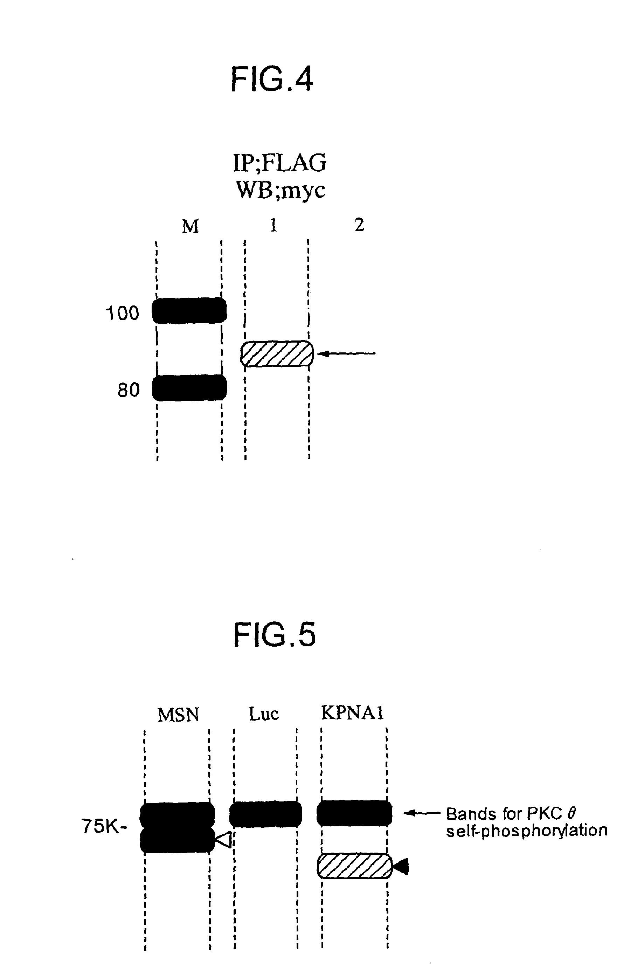

The result is shown in Figure 1. Figure 1 shows two electrophoresis images of right and left images. The following samples were applied to each lanes; M, 1 and 2. Figure 4 shows a diagram of a part of the left side of the electrophoresis of Figure 1.
Lane M: Molecular weight marker
Lane 1: Immunoprecipitated sample with anti FLAG antibody extracted from cell transfected by the combination 1.
Lane 2: Immunoprecipitated sample with anti FLAG antibody extracted from cell transfected by the combination 2. The right side electrophoresis image (WB; myc) shows that the FLAG-KPNA1 is immunoprecipitated (arrow). The left side electrophoresis image (WB; FLAG) shows that PKC theta-myc-His was coprecipitated with immunoprecipitation of FLAG-KPNA1 (arrow in lane 1). Numeric values on the left of each lane of right and left sides are molecular weights (kDa) .

First, as shown in the right side image of Figure 1 (Western blotting with anti-FLAG M2 Monoclonal Antibody), bands corresponding to FLAG-KPNA1 (molecular weight of about 63 kDa) for both combinations 1 and 2 were detected, demonstrating the validity of the immunoprecipitation experiment. Secondly, as shown in the left side image of Figure 1 (Western blotting with c-Myc (9E10) Monoclonal Antibody), band corresponding to PKC theta-myc-His (molecular weight of about 85 kDa, arrow in lane 1) for combination 1 was detected. This demonstrates FLAG-KPNA1 formed complex with PKC theta-myc-His in cells and coprecipitated. This result confirmed that PKC theta binds with KPNA1 in cells.

(Example 2) Analysis of phosphorylation of KPNA1 by PKC theta
With confirmation of interaction of PKC theta and KPNA1, the in vitro kinase assay was performed to confirm experimentally whether KPNA1 would be a phosphorylation substrate of PKC theta.

2-1) A positive control and negative control of kinase assay of PKC theta
MSN is publicly known as a phosphorylation substrate of PKC theta (Salvatore F. P. et al., (1998) The Journal of Biological Chemistry 273, 13: 7594-7603). The mammalian cell expression plasmid of human MSN protein, MSN-V5-His/pcDNA3.1 (Invitrogen) was used as a positive control of kinase assay of PKC theta. With this plasmid, MSN can be expressed in cells as protein tagged with V5-His at C-terminus (hereinafter called MSN-V5-His).

On the other hand, luciferase was used as a negative control. pCMV Tag 2 control (Stratagene) was used for mammalian cell expression plasmid of luciferase. With this plasmid, luciferase can be expressed as protein tagged with FLAG at the N-terminus (hereinafter called FLAG-Luc).

2-2) in vitro kinase assay
The substrate proteins (FLAG-KPNA1, FLAG-Luc, MSN-V5-His) were expressed in cells, and collected as immunocomplex.

First, 4 x 10⁵ of HEK293T cells were seeded onto 6 cm dishes, incubated at 37°C/5% CO₂ for overnight, and transfected by using FuGENE(Roche Diagnostics). Transfected plasmids were 2 microlitters each of FLAG-KPNA1/pCMV, pCMV Tag2 control, MSN-V5-His/pcDNA3.1

After transfection, each protein was expressed transiently by further incubation at 37°C/5% CO₂ for two more days. Then the cells were washed with ice-cooled D-PBS (Invitrogen), and suspended in 0.5 ml of Cell Lysis Buffer (20 mM Tris-HCl, pH7.5/ 150 mM NaCl/ 1 mM Na₂EDTA/ 1 mM EGTA/ 1% Triton / 2.5 mM sodium pyrophosphate/ 1 mM beta-glycerophosphate/ 1 mM Na₃VO₄/ 1 microgram/ml Leupeptin/ 1 mM PMSF), and left to stand for 30 minutes on ice. Then the cell extract was obtained by collecting supernatant with centrifugation at 14 krpm for 10 minutes at 4°C. Ten microlitters of agarose conjugated normal mouse IgG (SantaCruz) was added to this collected cell extract, and mixed by inverting tube. The supernatant was collected by centrifugation (Pre-clean).

Ten microlitters of the anti-FLAG M2 agarose affinity gel (Sigma) was added onto the supernatant of FLAG-KPNA1 and FLAG-Luc, and 10 microlitters of the anti-V5 agarose affinity gel (Sigma) was added onto the supernatant of MSN-V5-His, and mixed by inverting tubes for 2 hours at 4°C. The agarose was collected by centrifugation. The agarose was washed with 0.5 ml of Cell Lysis Buffer twice, and then with kinase buffer (25mM Tris-HCl pH7.5/ 5 mM beta-glycerophosphate/ 2 mM DTT/ 0.1 mM Na₃VO₄/ 10 mM MgCl₂) twice.

To the substrate protein collected by the above procedures, 24.5 microlitters of reaction mixture (the above kinase buffer plus ATP [final concentration of 10 microMolar], MnCl₂ [final concentration of 2 microMolar], phosphatidyl serine [final concentration of 5 microlitters/ml, phosphatidyl glycerol [final concentration of 0.2 mg/ml] and 5 microCi of gamma³²P-ATP) and 0.5 microlitter of purified PKC theta (Upstate) [equivalent to about 300 ng]. This mixture was reacted at 30°C for 30 minutes. Then SDS sample buffer was added and the mixture was boiled for 5 minutes. These samples were separated by SDS PAGE, and the X-ray film was exposed to the gel. The presence or absence of the substrate-specific bands was detected.

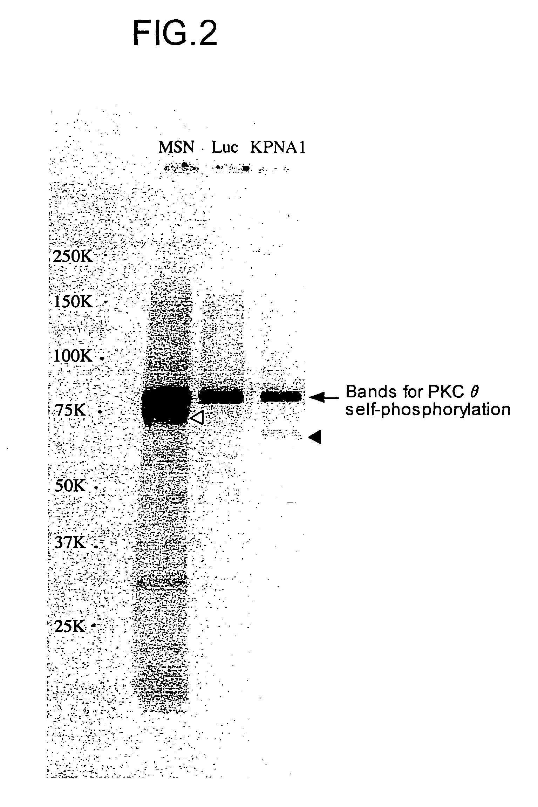

The result is shown in Figure 2. Figure 5 shows the diagram of the part of the electrophoresis image of Figure 2. The positive control MSN is phosphorylated by PKC theta (outlined arrowhead), the negative control luciferase is not phosphorylated by PKC theta. Under the same condition, KPNA1 is phosphorylated by PKC theta (black arrowhead). The arrow shows self-phosphorylation of PKC theta. The numeric values of the left side of the figure represent molecular weight of the molecular-weight marker (kDa) .

The self-phosphorylated band of PKC theta (molecular weight of about 83 kDa) was commonly observed in each lane. Since the positive control MSN contains phosphorylated band (molecular weight of about 71 kDa), and the negative control Luc does not contain phosphorylated band (molecular weight of about 62 kDa), the validity of the experiment system was demonstrated. Under this condition, phosphorylated band of KPNA1 (molecular weight of about 63 kDa) was observed. Thus, KPNA1 was confirmed to be a phosphorylation substrate of PKC theta.

(Example 3) Analysis of interaction of KPNA1 and NF-kappaB
The in vivo binding assay was performed to confirm experimentally whether KPNA1 and NF-kappaB interact or not.

3-1) Construction of expression plasmids of p50 and p60 which constitute NF-kappaB
It is publicly known that human NF-kappaB consists of heterodimer, p50 and p65. It is also known that human NF-kappaB is initially expressed as precursor protein p150 (NCBI: accession number AAA36361), and then digested between 436th methionine and 437th aspartic acid in cell, producing p50.

The cDNA coding amino acid sequence for human p50 was obtained by performing PCR with human skeletal muscle cDNA library (Takara Bio) as a template. The sequence of primers (p50-N and p50-C) used in PCR is shown in Sequence ID numbers 5 and 6 respectively in Sequence List. The sequence of p50-C is designed that the termination codon TAA is added after the 436th methionine (ATG) of p105.

The cDNA coding amino acid sequence for human p65 (NCBI; accession number AAA36408) was obtained by performing PCR with human thymus cDNA library (Takara Bio) as a template. The sequence of primers (p65-N and p65-C) used in PCR to obtain p65, is shown in Sequence ID numbers 7 and 8 respectively in Sequence List. The resultant DNA fragment was inserted into mammalian cell expression vector pCMV Tag 2A (Stratagene). Thus, myc-p50/pCMV and myc-p65/pCMV, the plasmids which can express human p50 as protein tagged with myc at N-terminus (hereinafter called myc-p50) and human p65 as protein tagged with myc at N-terminus (hereinafter called myc-p65) respectively in mammalian cells were constructed.

3-2) In vivo binding assay of KPNA1 with p50 and p65
4 x 10⁵ of HEK293T cells were seeded onto 6 cm dishes, incubated at 37°C/5% CO₂ for overnight, and transfected by using FuGENE(Roche Diagnostics). At this time, 3 types of transfection was performed, with two micrograms each of myc-p50/pCMV and FLAG-KPNA1/pCMV (combination 1), 2 micrograms each of myc-p65/pCMV and FLAG-KPNA1/pCMV (combination 2), and as a negative control, 2 micrograms each of pCMV Tag control (Stratagene) and FLAG-KPNA1/pCMV (combination 3) .

In addition, pCMV Tag3 control is a plasmid which can express luciferase as protein tagged with myc at N-terminus.

After transfection, each protein was expressed transiently by further incubation at 37°C/5% CO₂ for two more days. Then the cells were washed with ice-cooled D-PBS (Invitrogen), and suspended in 0.5 ml of Cell Lysis Buffer (20 mM Tris-HCl, pH7.5/ 150 mM NaCl/ 1 mM Na₂EDTA/ 1 mM EGTA/ 1% Triton / 2.5 mM sodium pyrophosphate/ 1 mM beta-glycerophosphate/ 1 mM Na₃VO₄/ 1 microgram/ml Leupeptin/ 1 mM PMSF), and left to stand for 30 minutes on ice. Then the cell extract was obtained by collecting supernatant with centrifugation at 14 krpm for 10 minutes at 4°C. Ten microlitters of agarose conjugated normal mouse IgG (SantaCruz) was added to this collected cell extract, and mixed by inverting tube. The supernatant was collected by centrifugation (Pre-clean).

Ten microlitters of the anti-myc agarose conjugate (SantaCruz) was added onto the each supernatant (immunoprecipitation of myc-p50, myc-p65 and myc-Luc) and mixed by inverting tubes for 2 hours at 4°C. The agarose was collected by centrifugation. The agarose was washed with 0.5 ml of Cell Lysis Buffer 4 times. SDS sample buffer was added to the agarose and the mixture was boiled for 5 minutes. The supernatant was separated by SDS-PAGE. Immunoprecipitation of myc-p50, myc-p65, myc-Luc was confirmed by western blotting with c-Myc (9E10) Monoclonal Antibody (SantaCruz). And co-precipitation of FLAG-KPNA1 was confirmed by western blotting using anti-FLAG M2 Monoclonal Antibody (Sigma). ECL plus western blotting kit (Amersham biosciences) was used for detection.

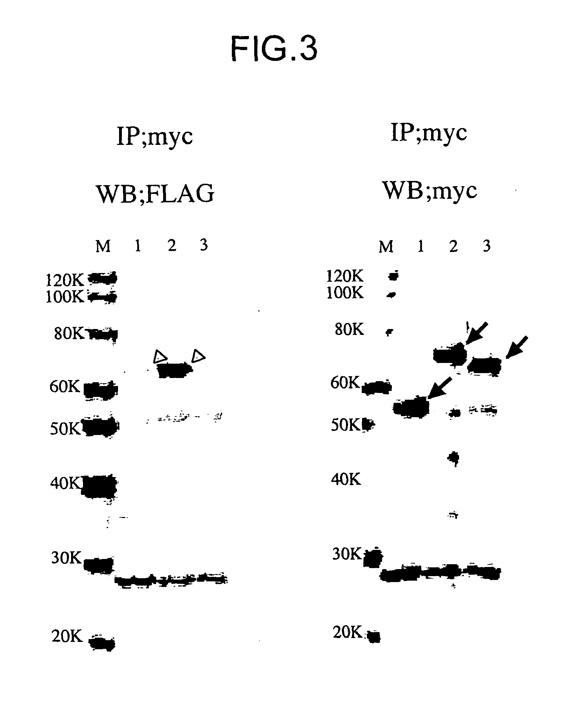

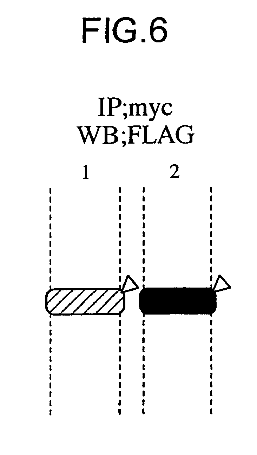

The result is shown in Figure 3. Figure 3 shows two electrophoresis images of right and left sides. The following samples were applied to each lanes; M, 1, 2 and 3. Figure 6 shows a diagram of a part of the left side of the electrophoresis of lanes 1 and 2.
Lane M: Molecular weight marker
Lane 1: Immunoprecipitated sample with anti myc antibody extracted from cell transfected by the combination 1.
Lane 2: Immunoprecipitated sample with anti myc antibody extracted from cell transfected by the combination 2.
Lane 3: Immunoprecipitated sample with anti myc antibody extracted from cell transfected by the combination 3.
The right side electrophoresis image (WB; myc) shows that the myc-p50 in lane 1, myc-p65 in p65, myc-Luc in lane 3 are immunoprecipitated (arrows in lanes 1, 2, and 3). The left side electrophoresis image (WB; FLAG) shows that KPNA1 was coprecipitated with immunoprecipitation of myc-p50 in lane 1 and myc-p65 in lane 2 (arrowheads in lanes 1 and 2). Numeric values on the left of each lane of right and left images are molecular weights (kDa).

First, as shown in the right side image of Figure 3 (Western blotting with c-Myc (9E10) Monoclonal Antibody), bands corresponding to myc-p50 (molecular weight of about 52 kDa), myc-p65 (molecular weight of about 65 kDa), myc-Luc (molecular weight of about 62 kDa) for combinations 1, 2 and 3 were detected, demonstrating the validity of the immunoprecipitation experiment. On the other hand, as shown in the left side image of Figure 3 (Western blotting with anti-FLAG M2 Monoclonal Antibody), band corresponding to KPNA1 (molecular weight of about 63 kDa) for combinations 1 and 2 was detected. And in the negative control combination 3, band corresponding to KPNA1 (molecular weight of about 63 kDa) was not detected. This demonstrates FLAG-KPNA1 formed complex with myc-p50 and myc-p65 in cells. This results confirmed that KPNA1 binds with NF-kappaB in cells.

(Example 4) Alteration of NF-kappa B's transcription activity due to phosphorylation of KPNA1 Study with the luciferases reporter assay was performed in order to examine whether or not the phosphorylation of KPNA by PKC theta affects transcription activity of NF-kappa B, in accordance with the following procedure.

4-1) Materials for Experiments 4-1-1) Host Cells
As the host cells for the reporter assay, Jurkat, Clone E6-1 (supplied from Dainippon Pharmaceutical Co., Ltd., referred to hereinbelow as the Jurkat cells) was used. The Jurkat cell is a cell strain established from T cells derived from a human with acute leukemia, and generally used as model cells for analyzing T cell activation in response to antigen recognition, in particular IL-2 gene expression. In the Jurkat cells, PKC theta, KPNA1 and NF-kappa B are expressed.

Construction of Expression Plasmids of Active PKC theta and Inactive PKC theta in Mammalian Cells PKC theta is composed of two domains that are the regulatory domain and the kinase domain. Usually, PKC theta is inactivated in terms of a phosphorylation enzyme in inactive T cells. This is due to pseudosubstrate region (a region having a sequence which is similar to the sequence of the substrate of PKC theta) in the regulatory domain which thrusts into the catalyst cleft of the kinase domain, to inhibit the kinase activity. However, when T cells are activated and produces lipid messengers such as diacylglycerol, such a messenger binds to the regulatory domain, which causes structural alteration of PKC theta, and releases the regulatory domain from the kinase domain. Consequently, PKC theta is activated as a phosphorylation enzyme, for transmitting signals of T cell activation.

PKC theta consists of 706 amino acid residues. Replacement of the 148th alanine which is in the pseudosubstrate region with glutamic acid results in the structural alteration which is equivalent to the aforementioned structural alteration. Therefore, PKC theta (referred to hereinbelow as PKC theta AE) is constantly activated as a phosphorylation enzyme. In Jurkat cells in which PKC theta AE is overexpressed, activation of IL-2 transcription can occur even without stimulation such as antigen recognition (Molecular and Cellular Biology 1996 Apr; 16 (4) : 1842-50.) .

It is known that mutation of the 409th lysine in PKC theta kinase domain by replacing the lysine residue with arginine results in inactivation thereof in terms of the phosphorylation enzyme. The lysine residue plays a role to direct the orientation of gamma phosphate group of the bound ATP to hydroxyl group in the substrate to be phosphorylated. It is known that, therefore, PKC theta having this mutation (referred to hereinbelow as PKC theta KR) can not smoothly pass the phosphate group to the substrate and is thus inactive in terms of a phosphorylation enzyme; and that Jurkat cells in which PKC theta KR is overexpressed do not activate IL-2 transcription even if stimulation such as antigen recognition is applied, regardless of existence of endogenic PKC theta (Molecular and Cellular Biology 1996 Apr;16(4) : 1842-50.)

In the present experiment, plasmids each expressing any one of PKC theta AE and PKC theta KR were produced on the basis of the aforementioned findings, since transcription activity of NF-kappa B has to be measured both in the state wherein KPNA1 is phosphorylated by PKC theta and in the state wherein KPNA1 is not phosphorylated.

Construction of plasmids each expressing any one of PKC theta AE and KR was performed with PKC theta-myc-His/pcDNA 3.1 which was also used in "In vivo binding assay of PKC theta and KPNA1". Two types of the plasmids were produced: one was produced by introducing mutation into DNA encoding wild-type PKC protein with QuickChange Site-Directed Mutagenesis Kit (Stratagene) to replace 148th alanine in the amino acid sequence with glutamic acid (referred to hereinbelow as PKC theta AE-myc-His/pc DNA 3.1); and the other was produced by replacing the 409th lysine with arginine (referred to hereinbelow as PKC theta KR-myc-His/pc DNA 3.1). The amino acid sequence of PKC theta AE is shown in SEQ ID NO:9,and PKC theta KR in SEQ ID NO:10.

Subsequently, for stable expression of PKC theta AE and KR in mammalian cells, the expression vector was changed from pcDNA3.1/myc-His to pCI vector (Promega). This vector contains artificially-produced intron positioned downstream of the enhancer/promoter of the human cytomegalovirus, which makes expression of the gene introduced downstream thereof more stable and high level. Each of PKC theta AE-myc-His/pcDNA3.1 and PKC theta KR-myc-His/pcDNA3.1 was treated with restriction enzymes Kpn I and Pme I to produce DNA fragements encoding PKC theta AN or KR with c-terminal myc-His tag, which were then incorporated into pCI vector between Kpn I site and Pme I site thereof. Thus, PKC theta AE-myc-His/pCI and PKC theta KR-myc-His/pCI were obtained. With these plasmids, PKC theta AE and KR with myc-His tag at the C-terminus thereof are each expressed in Jurkat cells.

4-2) Transfection and Reporter Assay As the reporter plasmid for detecting NF-kappa B transcription activity, pNF-kappa B-Luc (Stratagene) was employed. In this plasmid, an enhancer sequence (5'-TGGGGACTTTCCGC-3', SEQ ID NO: 11), which is necessary for NF-kappa B to work as a transcription activator factor, is repeatedly inserted five times upstream of DNA encoding firefly luciferase. That is, the reporter plasmid pNF-kappa B-Luc contains the sequence for region to which NF-kappa B binds (NF-kappa B binding region), and the firefly luciferase gene, acts as a reporter, downstream of the NF-kappa B binding region. Detection of the firefly luciferase activity shows that NF-kappa B binds to NF-kappa B binding region and acts as the transcription activator. As the internal standard, phRL-TK (Promega) was employed. This is a reporter plasmid containing a promoter sequence of thymidine kinase of herpes simplex, and a Renilla luciferase inserted downstream thereof.

As the plasmid capable of expressing KPNA1 in mammalian cells, FLAG-KPNA1/pCMV, which was also used in "In vivo binding assay of PKC theta and KPNA1" was employed.

In order to conform the total DNA amount upon transfection, pCI vector which was used for incorporating PKC theta AE and KR, and pCMV Tag2 for incorporating KPNA1 were used. As NF-kappa B, those inherently existed in the cells were used.

1 x 10⁵ of Jurkat cells were inoculated in 12-well plate and cultivated for overnight. Then transfection was performed with FuGENE6 (manufactured by Roche Diagnostics). As the plasmid DNA's for transfection, 190ng of pNF-kappa B-Luc, 10ng of phRL-TK, 400ng of either one of PKC theta AE-myc-His/pCI or PKC theta KR-myc-His/pCI, and 400ng of the combination of FLAG-KPNA1/pCMV and pCMV Tag2 were used, the total of which amounts up to 1 microgram. Variations of amount of FLAG-KPNA1/pCMV to be added were made at 0, 100, 200, 300, and 400 ng. When examining the effect of 400ng FLAG-KPNA1/pCMV only, 400ng of pCI vector was added.

After transfection, cultivation was further performed for 48 hours. With Dual-Luciferase Reporter Assay System(manufactured by Promega), firefly luciferase activity and Renilla luciferase activity were measured. The firefly luciferase activity was divided by Renilla luciferase activity, to calculate offset values. The offset value obtained by transfection with 400ng of PKC theta KR-myc-His/pCI and 400ng pCMV Tag2 being 1, relative values of the offset values of other combinations were calculated, to quantify the alteration of firefly luciferase expressed depending on the transcription activity of NF-kappa B. The experiments were performed six times independently, and the average and standard deviation were also calculated.

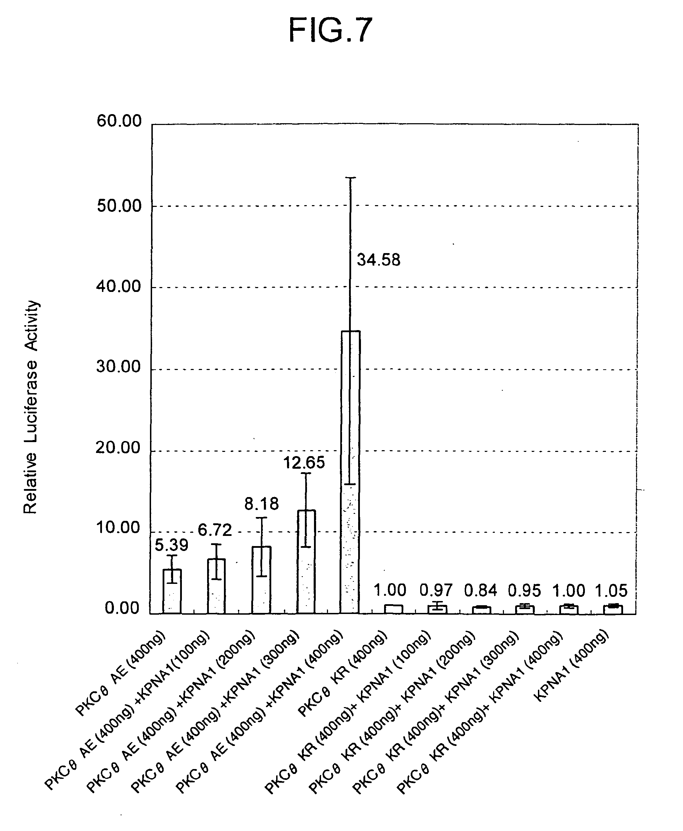

Results:
The graph of experimental results of averages with standard deviation is shown in Fig. 7.

Single expression of PKC theta AE which was activated as the phosphorylation enzyme results in six times higher NF-kappa B transcription activity than that with PKC theta AE alone, which proves adequacy of the experimental system. It is also demonstrated that co-expression of PKC theta AE and KPNA1 results in elevation of transcription activity of NF-kappa B as the expression of KPNA1 increases. The expression was further elevated to 6.4 times that of PKC theta AE alone. These phenomena were not observed at all with co-expression of PKC theta KR which is inactivated as the phosphorylation enzyme together with KPNA1, and expression of KPNA1 alone.

From the aforementioned results, it was confirmed that NF-kappa B's activity as the transcription activator is enhanced as a result of KPNA1's binding to PKC theta and phosphorylation thereof by PKC theta.

On the basis of the results of Examples 1 to 4, it is suggested that one of pathways for IL-2 expression may be as follows: Firstly, KPNA1, the protein for transporting transcription factors into nucleus, is phosphorylated. The phosphorylation of KPNA1 leads to efficient complex formation with NF-kappa B. NF-kappa B is then transported into the nucleus in a form of this complex. It is considered that there may be the pathway in which the NF-kappa B transported into the nucleus promotes transcription of IL-2 gene.

### [Industrial Availability]

The present invention is applicable in biotechnology related industries. The present invention is suitable for use in medicine/biological reagents developing and manufacturing industries.

### [Sequence List Free Text]

Sequence ID Number 1; primer (theta-N)
Sequence ID Number 2; primer (theta-C)
Sequence ID Number 3; primer (KPNA1-N)
Sequence ID Number 4; primer (KPNA1-C)
Sequence ID Number 5; primer (p50-N)
Sequence ID Number 6; primer (p50-C)
Sequence ID Number 7; primer (p65-N)
Sequence ID Number 8; primer (p65-C)
Sequence ID Number 9; amino acid sequence of PKC theta AE
Sequence ID Number 10; amino acid sequence of PKC theta KR
Sequence ID Number 11; enhancer sequence of NF-kappa B

## Claims

1. An inhibitor of protein-protein interaction inhibiting interaction between PKC theta and KPNA1.

2. An inhibitor of protein-protein interaction inhibiting interaction between KPNA1 and NF-kappa B.

3. An inhibitor of interaction between PKC theta and KPNA1, said inhibitor being obtained by examining whether the interaction between PKC theta and KPNA1 occurs in the presence of PKC theta, KPNA1, and candidate compounds under the conditions allowing interaction between PKC theta and KPNA1, and selecting a candidate compound showing inhibition of the interaction.

4. The inhibitor as set forth in claim 3, wherein said PKC theta is a kinase having a constant phosphorylation activity.

5. An inhibitor of interaction between KPNA1 and NF-kappa B, said inhibitor being obtained by examining whether the interaction between KPNA1 and NF-kappa B occurs in the presence of KPNA1, NF-kappa B, and candidate compounds under the conditions allowing interaction between KPNA1 and NF-kappa B, and selecting a candidate compound showing inhibition of the interaction.

6. A method for detecting an inhibitor of interaction between PKC theta and KPNA1, said method comprising the step of examining whether the interaction between PKC theta and KPNA1 occurs in the presence of PKC theta, KPNA1, and candidate compounds under the conditions allowing interaction between PKC theta and KPNA1, to detect a candidate compound showing inhibition of the interaction as the inhibitor.

7. The inhibitor as set forth in claim 6, wherein said PKC theta is a kinase having a constant phosphorylation activity.

8. A method for detecting an inhibitor of interaction between KPNA1 and NF-kappa B, said method comprising the step of examining whether the interaction between KPNA1 theta and NF-kappa B occurred in the presence of KPNA1, NF-kappa B, and candidate compounds under the conditions allowing interaction between KPNA1 and NF-kappa B, to detect a candidate compound showing inhibition of the interaction as the inhibitor.

9. A detection kit for an inhibitor of interaction between PKC theta and PKNA1, said kit comprising PKC theta supplying sample and KPNA1 supplying sample.

10. The detection kit for an inhibitor as set forth in Claim 9, wherein said PKC theta supplying sample is a vector containing polynucleotide coding PKC theta, and said KPNA1 supplying sample is a vector containing polynucleotide coding KPNA1.

11. The kit as set forth in claim 9 or 10, wherein said PKC theta is a kinase having a constant phosphorylation activity.

12. A detection kit for an inhibitor of interaction between KPNA1 and NF-kappa B, said kit comprising KPNA1 supplying sample and NF-kappa B supplying sample.

13. The detection kit for an inhibitor as set forth in Claim 12, wherein said KPNA1 supplying sample is a vector containing polynucleotide coding KPNA1, and said NF-kappa B supplying sample is a vector containing polynucleotide coding NF-kappa B.

14. A method for developing a medicine **characterized by** targeting the interaction between PKC theta and KPNA1 for drug discovery.

15. A method for developing a medicine **characterized by** targeting the interaction between KPNA1 and NE-kappa B for drug discovery.
